(19) **Europäisches Patentamt**
**European Patent Office**
**Office européen des brevets**

(11) **EP 3 332 031 B1**

(12) **EUROPEAN PATENT SPECIFICATION**

(45) Date of publication and mention
of the grant of the patent:
**08.04.2020 Bulletin 2020/15**

(21) Application number: **16767357.3**

(22) Date of filing: **04.08.2016**

(51) Int Cl.:
***C12Q 1/6883*** (2018.01)

(86) International application number:
**PCT/IB2016/054715**

(87) International publication number:
**WO 2017/021926 (09.02.2017 Gazette 2017/06)**

(54) **SNP RS12603226 AS A PREDICTIVE MARKER FOR NAFLD**

SNP RS12603226 ALS PRÄDIKTIVER MARKER FÜR NAFLD

POLYMORPHISME D'UN SEUL NUCLÉOTIDE (SNP) RS12603226 EN TANT QUE MARQUEUR
PRÉDICTIF POUR LA NAFLD (STÉATOSE HÉPATIQUE NON ALCOOLIQUE)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **05.08.2015 IT UB20152872**

(43) Date of publication of application:
**13.06.2018 Bulletin 2018/24**

(73) Proprietor: **Orga Bio Human S.r.l.**
**00144 Roma (RM) (IT)**

(72) Inventors:
• **ROSATI, Paolo**
**00144 Roma (IT)**
• **ROSATI, Davide**
**00144 Roma (IT)**
• **MARINI, Rita**
**00126 Roma (IT)**

(74) Representative: **Raimondi, Adriana et al**
**Cavattoni - Raimondi,**
**Viale dei Parioli, 160**
**00197 Roma (IT)**

(56) References cited:
**US-A1- 2010 056 384**

• **"Reference SNP (refSNP) Cluster Report:**
**rs12603226", , 21 February 2004 (2004-02-21),**
**pages 1-1, XP055249602, Retrieved from the**
**Internet:**
**URL:http://www.ncbi.nlm.nih.gov/projects/S**
**NP/snp_ref.cgi?rs=12603226 [retrieved on**
**2016-02-12]**
• **NOBILI, V., ET AL: "A 4-Polymorphism Risk Score**
**Predicts Steatohepatitis i Children With**
**Nonalcoholic Fatty Liver Disease", JOURNAL OF**
**PEDIATRIC GASTROENTEROLOGY AND**
**NUTRITION, vol. 58, no. 5, 1 April 2014**
**(2014-04-01), pages 632-636, XP002754259,**
• **Bernd Schnabl ET AL: "Increased expression of**
**Zinc finger protein 2687 in non-alcoholic fatty**
**liver disease", Int J Clin Exp Pathol, 1 January**
**2011 (2011-01-01), pages 661-666, XP055249890,**
**Retrieved from the Internet:**
**URL:http://www.ncbi.nlm.nih.gov/pmc/articl**
**es/PMC3209606/pdf/ijcep0004-0661.pdf**
**[retrieved on 2016-02-15]**
• **NAGA CHALASANI ET AL: "Genome-Wide**
**Association Study Identifies Variants Associated**
**With Histologic Features of Nonalcoholic Fatty**
**Liver Disease", GASTROENTEROLOGY, vol. 139,**
**no. 5, 1 November 2010 (2010-11-01), pages**
**1567-1576.e6, XP055249895, PHILADELPHIA, PA**
**ISSN: 0016-5085, DOI:**
**10.1053/j.gastro.2010.07.057**

## Description

### Field of the Invention

This invention relates to the use or SNPs (Single Nucleotide Polymorphisms) for predicting the risk of the development of non-alcoholic fatty liver disease (NAFLD) and diseases associated therewith.

### Prior art

Considered in the past to be a benign condition, in reality NAFLD is now considered to be one of the greater causes of organ damage and it has been estimated that it will be one of the major indications for liver transplants in the next few years (Dutkowski, P. et al., Gastroenterology 2015 Feb;148(2):307-23.). NAFLD can in fact follow a developmental course progressing through an inflammatory stage, referred to as non-alcoholic steatohepatitis (NASH) to fibrosis, cirrhosis and possibly liver cancer (Brunt, Nat. Rev. Gastroenterol. Hepatol. 2010;7:195). NAFLD, which is characterised by an excessive build-up of triglycerides in the liver, is a multi-factor disease comprising different forms of liver damage, such as simple steatosis or steatohepatitis or NASH (non-alcoholic steatohepatitis), which may be associated with fibrosis (Brunt, Nat. Rev. Gastroenterol. Hepatol. 2010;7:195.). For a detailed description of the histological characteristics of NAFLD, see Brunt, 2010 (above).

Many clinical studies have demonstrated that both environmental factors (such as an inappropriate lifestyle) and genetic factors are among the greatest risk factors for NAFLD (Anstee, Q.M., Day, C.P., Nat. Rev. Gastroenterol. Hepatol. 2013 Nov; 10(11):645-55.).

NAFLD is the predominant cause of chronic liver disease in western countries, and it is expected that it will become the main cause of cirrhosis making it necessary to resort to liver transplants in the next twenty years. Most patients with NAFLD also present with metabolic risk factors, such as, for example, obesity, Type 2 diabetes and dyslipidaemia. The accumulation of visceral fat, the presence of metabolic syndrome and insulin resistance are other major risk factors for the development of NAFLD (Masuoka, H.C. and Chalasani, No. Ann. No. Y. Acad. Sci. (2013) 1281: 106-12). Only a proportion of patients who suffer from hepatic steatosis (around 20%) develop NASH over time. The latter are at high risk of developing cirrhosis and liver cancer (Masuoka, H.C. et al., (2013) see above). In almost all cases NAFLD is an asymptomatic and silent disease. It is in fact often diagnosed through the presence of anomalous liver biochemistry tests, generally performed as routine checks.

Because of these characteristics NAFLD is often overlooked or underestimated, and the often fortuitous diagnosis is made when the liver is already compromised and histological features of NASH are obvious following liver biopsy (Brunt, 2010, see above; Masuoka, H.C. et al., 2013 see above). Proper diagnosis of NASH and the presence of cirrhosis and inflammation are only ensured by histological analysis of a liver biopsy. However resort to biopsy is difficult because of its high cost and above all because of its invasive nature; in addition to this, the operator's skill and experience are an important variability factors for correct performance of the biopsy, sampling and final diagnosis (Masuoka, H.C. et al., (2013) see above) and only a miniscule part (approximately 1/50,000) of the liver is sampled with biopsy sampling, contributing to the extreme criticality of this procedure. Research in recent years has therefore focused on the identification of biomarkers for the early diagnosis and prognosis of NAFLD (Fitzpatrick, E. and Dhawan, A. (2014) World J. Gastroenterol. 20:10851-10863).

By definition a marker is such if it is sufficiently sensitive to identify sick patients, if it is sufficiently specific to distinguish the latter from healthy patients with adequate efficiency, if its presence is highly correlated with the probability of the development of a disease, if the analysis of its levels is not excessively expensive or difficult to perform, and, above all, if such analysis is reproducible. Finding non-invasive, diagnostic and prognostic markers capable of providing information on NAFLD and the histological features associated with this disease is of great clinical significance. (Fitzpatrick, E. and Dhawan, A. (2014), see above).

Recently two types of non-invasive markers which can have a significant impact on clinical practice have come to light: the first are systemic markers strictly correlated with the pathogenesis of the disease (e.g.: hyaluronic acid and cytokeratin 18) (Fitzpatrick, E. and Dhawan, A. (2014), see above); the second are genetic markers. In fact one method for evaluating the risk of the occurrence and development of NAFLD in an individual comprises detecting the allele status of one or more polymorphisms in a nucleic acid sample (Nobili, V. et al.; J. Pediatr. Gastroenterol. Nutr.; 2014 May; 58(5): 632-6).

Polymorphisms are variations in DNA sequences which are present in a population with a frequency of more than 1%; in particular single nucleotide polymorphisms or SNP are, by definition, "variants" of a single DNA base present in the population (Gusella , J.F.; Annual Review of Biochemistry; July 1986; Vol. 55:831-854). These can be found at any point in the genome, in both the coding and non-coding portions, but can nevertheless have a protective and regulating role. These variants may be implicated in the development of diseases, response to drugs, response to infections, exposure to chemical agents, etc.

When considered individually, SNP generally have a variable predictive "weight" associated with their statistical correlation with the disease, but by analysing several SNPs in the genome of one individual they can provide greater predictive value, characterised by greater specificity and sensitivity, above all in complex diseases such as NAFLD and cardiovascular diseases (Vasan, R.S., Circulation 2006 May 16; 113(19):2335-62.).

Epidemiological, family and twin studies have demonstrated that some SNPs (genetic variants) have a part to play in both the severity of the disease and the progression from NAFLD to NASH (Dongiovanni, P. et al. Genetic Predisposition in NAFLD and NASH: Impact of Severity of Liver Disease and Response to Treatment; Curr. Pharm. Des. 2013 Sept.; 19(29): 521495238).

The SNPs hitherto used for the prediction of NAFLD, KLF-6 rs3750861, PNPLA-3 rs738409, LIPIN-1 rs13412852, SOD-2 rs4880 (Nobili. V. et al. J. Pediatr. Gastroenterol. Nutr. 2014 May;58(5):632-6), and TM6SF2 rs58542926 (Sookoian, S. et al., Hepatology. 2015 Feb;61(2):515-25) have a variable correlation with the disease, which is not yet sufficiently strict, leaving open the need to find further SNP that are predictive of NAFLD with a stronger correlation with the disease, so that existing prediction tools can be improved.

Hitherto the search for relevant SNPs for predisposition to NAFLD has been directed towards genes that are functionally involved in the molecular pathogenesis of the disease. But, as is known from other diseases, there may be variant alleles in exonic or intronic regions which are still not known but are capable of facilitating the occurrence of a NAFLD phenotype.

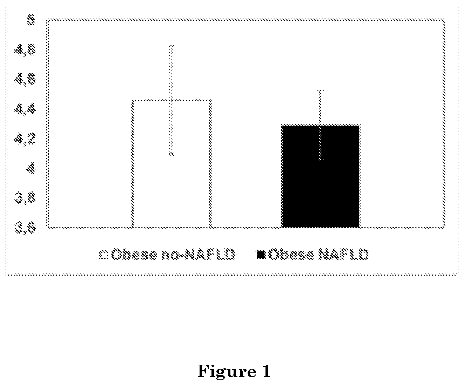

Recently the role of a protein belonging to the "Kruppel-like zinc finger" proteins (Schnabl, B. et al. Int. J. Clin. Exp. Pathol. 2011;4(7):661-6) known as ZNF267, has been demonstrated. This study reported an increased expression of the protein ZNF267 in the liver of patients suffering from NAFLD in comparison with patients having a healthy liver. The role of the ZNF proteins in NAFLD has hitherto been little described, partly because little is also known about their general function. When research was carried out on databases reporting gene expression profiles in obese patients affected by NAFLD (Www.ncbi.nlm.nih.gov/geoprofiles; www.genecards.org) data emerged regarding the coding transcripts for six ZNF proteins. One transcript in particular is reduced in obese patients affected by NAFLD (Figure 1).

This transcript codes for the protein ZNF624 whose RNA is expressed in various tissues, including the liver (http://www.ncbi.nlm.nih.gov/geo/tools/profileGraph.cgi?ID=GDS4881:801_3035). Different allele variants in regions codifying the gene for ZNF624 have been identified and studied, but as far as the inventors are aware no studies relating to the allele frequencies of SNP rs12603226, which is found on chromosome 17 in position 16626253 in intron region NM020787.3 within the ZFN624 gene, in the general population have been reported.

As far as the inventors are aware there is no document in the literature describing the function of this protein or any study reporting allele frequencies of possible SNPs present in its coding gene in the general population. (Nobili, V. et al., J. Pediatr. Gastroenterol. Nutr. 2014 May; 58(5): 632-6).

Summary of the Invention

It has now been found that the SNP rs 12603226 of the ZNF624 gene, when present with the homozygous TT genotype, has a very significant correlation with NAFLD, which is stronger than the correlation with the disease hitherto described for known SNPs (such as e.g. KLF-6 rs3750861 PNLA-3 rs738409 LIPIN-1 rs13412852 SOD-2 rs4880). The homozygous TT status has in fact a very much higher incidence in individuals with NAFLD in comparison with healthy individuals in our study.

The object of this invention therefore relates to an SNP in rs12603226 (chromosome 17 in position 16626253 in the NM020787.3 intron region within the ZFN624 gene. (http://www.ncbi.nlm.nih.gov/projects/SNP/snp_ref.cgi?rs=12603226) in which the A base is substituted by a T base. This variation, when homozygous in a individual is associated with a high risk of developing NAFLD.

A further object of this invention is a method for predicting an increased risk of non-alcoholic fatty liver disease (NAFLD) in an individual, which comprises the stage of genotyping the SNP rs12603226 in a nucleic acid obtained from a biological sample from the individual and in which the presence of the homozygous TT allele indicates an increased risk of developing NAFLD.

Further objects will be apparent from the detailed description of the invention.

Brief Description of the Figures

Figure 1. Expression of ZNF624 mRNA in the liver in obese individuals without NAFLD and in obese individuals with NAFLD. The ZNF624 mRNA in obese individuals without NAFLD and in obese individuals with NAFLD is shown along the Y axis.

Detailed Description of the Invention

The following definitions are provided within the scope of this invention.

By **polymorphism** is meant the occurrence of two alternative genome sequences between two or more different genomes or individuals.

By **polymorphic site** is meant the locus in which the variation is to be found. The polymorphic site has at least two alleles, each present with a specific frequency in a selected population.

By **allele** is meant one of the alternative forms which a nucleotide sequence may adopt at the same chromosome site.

By **Single Nucleotide Polymorphism (SNP)** is meant a polymorphic site comprising a single nucleotide which has two alternative alleles.

By **genotype** is meant the description of the allele status of a genome sequence in an individual.

By **primer** is meant an oligonucleotide of variable length which acts as a starting point for the synthesis of nucleic acid in a PCR (Polymerase Chain Reaction).

By **probe** is meant an oligonucleotide of variable length which hybridises with a complementary target nucleic acid.

By **target sequence** is meant a region of nucleic acid which has to be analysed and comprises the polymorphic site of interest.

By **genotyping** is meant the process of determining the allele status of an SNP in an individual.

By **allele frequency** is meant measuring the relative frequency of an allele in the population expressed as a proportion or percentage value.

By **genotype frequency** is meant the frequency or proportion of genotypes present in a population.

By **ballooning** is meant the swelling of hepatocytes with clear rarefied cytoplasm, hyperchromatic nucleus, lobular inflammation characterised by infiltrations of inflammatory cells such as lymphocytes and eosinophils, and fibrosis caused by the presence of collagen around the hepatocytes.

By **BMI (Body Mass Index)** is meant a general evaluation of body mass as an indirect index of adiposity. BMI, expressed as $kg/m^2$, is calculated by dividing the weight, expressed in kg, by the square of height expressed in m. BMI is interpreted on the basis of criteria defined by the World Health Organisation (http://www.who.int/en/) which has established classes for BMI:

Severely underweight (BMI < 16.00), Underweight (16.00 < BMI <18.49), Normal weight (18.50 < BMI < 24.99), Overweight (25.00 < BMI < 29.99), Class 1 obesity (30.00 < BMI < 34.99), Class 2 obesity (35.00 < BMI < 39.99), Class 3 obesity (BMI $\geq$ 40.00).

In infancy and adolescence BMI is marked by appreciable variability, associated above all with sex and age, and it is preferable to use the graph of percentiles as a reference (Cole et al., BMJ 2000 May 6;320(7244): 1240-3) and the following subdivision into classes: underweight (<5th percentile), normal weight (>5th, <85th), at risk of overweight (>85th, <90th), overweight/obesity (>90th).

The inventors have now investigated different variant alleles in coding and non-coding regions of the ZNF624 gene (http://www.ncbi.nlm.nih.gov/gene/?term=ZNF624+human), located on chromosome 17, and have surprisingly found a strong correlation between one of the polymorphisms present in the ZNF624 genome sequence and NAFLD and its progress. Specifically the inventors have analysed the allele frequencies of SNP rs12603226 (http://www.ncbi.nlm.nih.gov/ snp/?term=rs12603226) found in position 16626253 in intron region NM020787.3 of the ZFN624 gene.

As is known to those skilled in the art, an SNP may have two different alleles, each of which corresponds to a nucleotide present in the DNA (A, G, C, T,), in the case of SNP rs12603226 an alternative between an Adenine (A) or a Thymine (T). As far as the inventors are aware there are no studies on the allele frequencies of the abovementioned SNP.

The process of determining a specific nucleotide in a specific position of the genome can be performed by different methods known to those skilled in the art (Chen, X., Sullivan, P.F., Pharmacogenomics J. 2003;3(2):77-96; Marnellos, G., Curr. Opin. Drug Discov. Devel. 2003 May;6(3):317-21) (Chen et. Al., Pharmacogenomics J. 2003 April;3(2):197-205).

TABLE 1 below lists some methods commonly used for the genotyping of SNPs by way of a non-limiting example.

**TABLE 1.**

| TaqMan Samples | US Pat. No. 5,210,015; 5,538,848; 5,801,115 |
| --- | --- |
| Samples using molecular beacons and other variants; scorpions; FRET; or High Melt Resolution, etc. | US Pat. No.5,118,801; US Pat. No. 5,866,336; 6,117,635; US Pat. No. 5,118,801; US Pat. No. 20090197254; US Pat. No. 20050042168 A1; |

(continued)

| PCR with Reverse Hybridisation (Reverse Dot-Blot) | Pat. WO1993009245A1 |
|---|---|
| Nucleic acid arrays | US Pat. No.7904250; Barisic I et al.; Appl. Microbiol. Biotechnol. 2015 Jan;99(1):413-23 |
| Analyses (SSCP Single Strand Conformation Polymorphism) | Humphries et al.; R. Elles, ed., p.321-340; 1996 |
| DGGE (Denaturising gradient gel electrophoresis) | Wartell et al., Nucl. Acid Res. 18:2699-2706, 1990 |
| Extension of the primer | "Genetic Bit Analysis" method - WO92/15712 |
| Genetic analysis mediated by ligases/ polymerases and variants and correlated methods | US Pat. No. 5,679,524; WO91/02087, WO90/09455, WO95/17676, US Pat. No. 5, 302, 509 E 5,945,283; US Pat. No. 5,605,798; WO 93/22456; Wallace et al. in WO89/10414 |
| OLA (OLA TEST) Oligo Ligation Assay and variants | US Pat. No. 4,988,617; US Pat. No. 6,027,889; 6,268,148; 5494810; 5830711; 6054564; WO97/31256 and WO 00/56927 |
| Direct sequencing using different procedures and using the analysis of fragments | WO 94/16101; Bonneau, J., Hayden, M.; Methods Mol. Biol. 2014; Kuhn, D.N., Electrophoresis 2005 Jan;26(1): 112-25; |
| MLPA (Multiplex Ligation dependent Probe Amplification) | US Pat. 6955901; CA Pat. No. 2400240 |
| Pyrosequencing | Pat. WO2007002204A2; US Pat. N.7727723; |
| Next Generation Sequencing (NGS) Illumina; Life Technologies; Roche | Lan, J.H. et al., Hum. Immunol. 2015 Mar;76(2-3):166-75 Mikkelsen, M. et al., Forensic Sci. Int. Genet. 2014 Sept; 12: 30-7PLoS One. 2013 Jul24;8(7) Clarke, A.C. et al., BMC Genomics 2014 Jan 25; 15:68 |
| Strand Displacement Amplification (SDA) | US Pat. Nos.5,270184 and 5,422,252 |
| In Vitro Isothermal Amplification of DNA and its variants (LAMP - loop amplification) | Pat. WO00/28082; WO01/34790; WO 01/77317; US 8093030 |
| Isothermal Amplified Detection of DNA and RNA | Mol. Biosyst. 2014 May; 10(5):970-1003 |
| Linear Amplification (LLA) | US Pat. No. 6,027,923; WO1995003432 A1 |
| Transcription Mediated Amplification (TMA); NASBA (Nucleic Acid Sequence Based Amplification); self-sustained sequence replication | US Pat. No. 5,399,491; Pat. EP0629706B1; Pat. EP0572417A1 |
| RFLP (Restriction Fragment Length Polymorphism) polymorphism from the length of the restriction fragments. | Freitas, R.G., Campana, E.M., Pozzan, R., Brandão, A.A., Brandão, A.P., Magalhães, M.E., Silva, D.A., Arq. Bras. Cardiol. 2015 Jun;104(6):468-474. |
| Southern Blot | Kufe, D.W., Pollock, R.E., Weichselbaum, R.R. et al., editors. Hamilton (ON): BC Decker; 2003. |

The inventors have analysed the allele frequencies of SNP rs1263226. 400 biological samples deriving from 248 healthy individuals and 152 patients with histologically confirmed NAFLD were genotyped for SNP rs 1263226.

Genotyping was carried out using the TaqMan® 5' nuclease assay method, also known as TaqMan® allelic discrimination assay (Life-Technologies), following the manufacturer's instructions. The test makes it possible to discriminate between the two alleles, making use of the 5'-3' nuclease activity of TaqDNA polymerases to cause the release of allele-specific fluorescent probes from DNA.

Analysis of the genotypes (**TABLE 2**) reveals that the TT genotype has greater representation in individuals affected by NAFLD than in the population of healthy individuals. In fact, as frequency analysis of the genotypes demonstrates, the AT heterozygote is more frequent in the general healthy population while the TT homozygote is more frequent in individuals with NAFLD. The data deriving from analysis of the genotype frequencies observed (**TABLE 2**) indicates that presence of the homozygous T allele represents a risk factor for the disease and for the other co-morbidities associated with it.

Analysis of this SNP identifies individuals at genetic risk from the disease more accurately and with much higher correlation than the SNPs previously described.

For example SNP rs738409 present in the PNLPLA3 gene has hitherto been the most important polymorphism for predicting NAFLD; as reported in the literature (Valenti et al., Hepatology 2010 Apr;51(4):1209-17) the genotype considered to be predisposing (GG) has a frequency of 12%-14% in individuals with NAFLD in comparison with the general population, in which it is present in approximately 3% of cases.

In the case of SNP rs12603226 the inventors have surprisingly found that the predisposing genotype (TT) is present in 91% of cases in individuals with NAFLD, in comparison with the control population (approximately 7% of cases). The correlation (91%) between the predisposing genotype (TT) of SNP rs12603226 which is the subject of the invention and the disease is very much higher than the correlation between NAFLD and the rs738409 marker present in the PNLPLA3 gene (14%). The inventors have thus demonstrated that TT homozygosis for rs12603226 now represents a more reliable marker for prediction of the genetic risk of developing NAFLD than those currently used for predictive purposes.

A method for predicting predisposition to NAFLD has been developed on the basis of the results of the studies performed.

The method provides for the following fundamental stages:

- genotyping of SNP rs12603226 on a nucleic acid obtained from a biological sample from the individual;
- analysis of the genotype:

    TT allele homozygosis indicates an increased risk of developing NAFLD;
    AT allele heterozygosis indicates a lower risk of developing NAFLD.

Genotyping is a technique known to those skilled in the art.

The following examples are to be regarded as being illustrative of the invention and do not limit its corresponding scope.

Examples

*Characterisation of the population examined*

400 saliva samples, of which 152 samples were obtained from patients with NAFLD, confirmed by liver biopsy, and 248 samples obtained from healthy individuals were used for the genotyping of SNP rs12603226.

All individuals affected by NAFLD/NASH who proved positive for one or more tests for determining secondary causes of steatosis (e.g. drinking of alcohol, hepatitis, CMV, EBV, drugs affecting steatosis: valproate, amiodarone, prednisone, autoimmune diseases of the liver, etc.) were ruled out from the study.

Included in the study were individuals suffering from NAFLD aged between 6 and 14 years in the BMI>90th percentile (overweight/obesity). For histological characterisation (including steatosis, inflammation, ballooning of hepatocytes and fibrosis) reference was made to the NAFLD Scoring System of the NASH Clinical Research Network, sponsored by the National Institute of Health (Kleiner, D.E. et al., Hepatology 2005;41:1313-21). Age (age between 6 and 16 years; mean: 11) and BMI between the 5th and 84th percentile (normal weight) were considered in the case of healthy individuals.

**Extraction of DNA**

Before proceeding to genotype SNP rs12603226 (allele discrimination), DNA was extracted from biological samples using the QIAamp DNA Mini Kit (Qiagen, code: 51304), according to the manufacturer's instructions for removal from the buccal tampon.

The DNA so obtained was quantified using a NanoDrop (Thermo Scientific) spectrophotometer after selecting the application for nucleic acid and after cleaning the optical surface; a blank with 1.5 $\mu$l of sterile deionised water was first performed; subsequently 1.5 $\mu$l of DNA was extracted from each sample, evaluating both DNA concentration and purity using the 260/280 and 230/260 ratios. The samples were kept at - 20°C and subsequently used for the genotyping reaction.

*Procedure for Amplification and Detection of SNP rs12603226*

The TaqMan® 5' nuclease assay (Life Technologies) method was used to determine the allele status of SNP rs12603226. In short the two SNP alleles were amplified using pairs of specific primers for the region under investigation, and two TaqMan allele-specific probes intended to detect the target polymorphism (rs12603226 SNP A/T). The TaqMan probes contain a "reporter dye" (VIC® specific to the A allele; FAM™ specific to the ancestral T allele) on the 5' terminal part and a "quencher" (MGB Minor Binding Groove) on the terminal 3' part. During amplification each probe (specific allele with specific "reporter") was bound to one of the alleles. As amplification proceeded the Taq Polymerase cuts off the probe linked to the target, giving rise to the emission of fluorescence (which is recorded and interpreted by the software). The presence of VIC fluorescence alone indicates a homozygous status for the A allele; the presence of FAM alone indicates a homozygous status for the T allele; the presence of both VIC and FAM signals indicates the heterozygous status, that is the presence of both A and T alleles.

**Design of probes and Primers:**

The primers and probes were designated using the Custom TaqMan Design Tool (Life Technologies):

- Fluoresceinated VIC-MGB probe for detection of the **A** allele sequence (SEQ ID NO.1): CCCAGCCAAATTTATGA
- 6FAM-MGB fluoresceinated probe for detecting the **T** allele sequence (SEQ ID NO. 2): CCCAGCCAATTTTATGA
- FORWARD Primer (SEQ ID NO.3): CAGGCGTGAGCCACTGT
- REVERSE Primer (SEQ ID NO.4): GACAAATCATACTGCTTAAAAGTGGGTTT

The reaction was performed using 15 ng of genome DNA in a final volume of 12 µl, using the mixture provided by the manufacturer (TaqMan® Genotyping Master Mix-life Technologies code: 4371355) and the primers and probes described above. Amplification of the samples was performed using an HT 7900 Fast Real Time PCR System (Applied Biosystems) and the thermal protocol shown in the scheme below:

| | |
|---|---|
| Pre-PCR Read: (Hold Stage) | 60°C x 1 minute |
| Hold Stage: | 95°C x 10 minutes |
| Cycling Stage: | $\begin{bmatrix} 95°C \text{ x } 10 \text{ seconds} \\ 60°C \text{ x } 1 \text{ minute} \end{bmatrix}$ 40 cycles |
| Post-PCR: (Hold Stage) | 60°C x 1 minute |

The results obtained were analysed using ABI PRISM SDS Software version 2.2 (Applied Biosystems, Foster City, CA) and are shown in Table 2.

Statistical analysis

**TABLE 2** shows the genotype frequencies found by the analysis of SNP rs 12603226.

**TABLE 2**

| Genotype and Genotype frequency | AA (frequency) | AT (frequency) | TT (frequency) | Total |
|---|---|---|---|---|
| Healthy individuals | 0 (0) | 229 (0.9234) | 19 (0.0766) | 248 |
| NAFLD/NASH patients | 0 (0) | 13 (0.0855) | 139 (0.9145) | 152 |
| Total | 0 | 242 | 158 | 400 |

Analysing the genotype frequencies and proportions in the data from our study it is obvious that the TT genotype is correlated with NAFLD. In fact 91.45% of individuals with NAFLD/NASH (139/152) presented the TT genotype in comparison with the control group of healthy individuals (19/248; 7.66%), while only (13/152) 8.55% of individuals with NAFLD presented the AT genotype in comparison with the group of healthy individuals (229/248; 92.34%), confirming our hypothesis that the TT genotype of rs 12603226 of the ZNF624 gene is associated with NAFLD and the diseases correlated with it.

After genotyping, the $\chi^2$ Test and calculation of the Odds Ratio (OR) were used for statistical evaluation of the significance of the data obtained.

The $\chi^2$ Test was used to evaluate the association between the TT genotype and the development of NAFLD. In statistics the $\chi^2$ Test is used to test the hypothesis that there is at least one association between two groups, populations or criteria. The high value for the $\chi^2$ Test obtained in our study (**TABLE 3**) indicates that the results obtained are not due to randomness and that there is a strong association between the presence of the TT genotype and the development of NAFLD.

In statistics calculation of the Odds Ratio (OR) index is used in random-control studies to define the cause-effect ratio between two factors (for example: risk factor and development of a particular disease), to verify whether the presence of a risk factor or any other variable can cause a particular effect, establishing how many of the cases and controls are exposed to the presumed risk factor taken into consideration in the specific study. The frequency factor for the risk (presence of TT genotype) in NAFLD/NASH individuals in comparison with the frequency of that factor in the group of healthy individuals (control) was compared using the Odds Ratio (OR) calculation to verify the probability of the risk factor (TT genotype) being presented in individuals affected by NAFLD in comparison with the probability of the risk factor (TT genotype) being presented in "control" individuals.

An OR index having a value <1 indicates a negative association (that is the factor taken into consideration may be protective), while a value of OR >1 indicates a positive association (that is that the factor taken into consideration may result in development of the disease). The further the values depart from the value of 1 the stronger the association (in both cases). The high positive OR value obtained (OR=128.9; **TABLE 3**) shows that there is a strong statistical association between the presence of the TT genotype and NAFLD.

It is therefore possible to state that the data obtained through the $\chi^2$ Test and calculation of the Odds Ratio index are statistically significant, in that the p value or p-value for both is in both cases below 0.05, that is statistically significant. It is therefore possible to conclude that there is a strong association between the presence of the TT genotype and NALFD.

### TABLE 3: $\chi^2$ Test and ODDS RATIO (OR)

|  | TT | AT | Total |
|---|---|---|---|
| NAFLD/NASH Patients (Cases) | 139 | 13 | 152 |
| Healthy Patients (Control) | 19 | 229 | 248 |
| Total | 158 | 242 | 400 |

$\chi^2$ **value:** 276.8;
**p-value** < 0.0001
**Odds Ratio:** 128.9
**95% Confidence Interval:** 62 - 269
**p-value** < 0.0001

(Confidence Interval describes the uncertainty associated with a method of sampling. In statistics, when a parameter is estimated, the mere identification of a single value is often insufficient; therefore, in order to be able to estimate such a parameter a range of plausible values for that parameter which is defined as the **confidence interval** is used. The uncertainty of the estimate can be quantified by calculating confidence intervals. Altman defines the 95% interval as "the interval within which we can be certain 95% of the time that the value for the population falls within it").

N.B.: The OR (odds ratio), standard error and 95% CI (Confidence Interval., which describes the uncertainty associated with a method of sampling) were calculated according to Altman, 1991.

In conclusion, study of the allele status of rs 12603226 makes it possible to identify those individuals who are most at risk of developing NAFLD and diseases associated with it.

In particular, from our study of SNP rs12603226, it is clear that homozygous presence of the T allele (TT genotype) is closely correlated with the occurrence and progression of NAFLD. In fact the TT genotype was detected in 91.45% of individuals suffering from NAFLD. Thus study of the allele status of rs 12603226 can be used as a genetic marker for assessing the risk of the development and progress of NAFLD and diseases associated with it.

## Claims

1. A method for predicting an increased risk of non-alcoholic fatty liver disease (NAFLD) in an individual which comprises the stage of genotyping SNP rs12603226 carried out on a nucleic acid obtained from a biological sample from the individual and in which the presence of allelic homozigosity TT indicates an increased risk of the development of NAFLD.

2. Method according to claim 1, which further comprises the stage of genotyping one or more SNPs associated with NAFLD, selected from: KLF-6 rs3750861 in which the predisposing genotype is CC; PNPLA-3 rs738409 in which the predisposing genotype is CG or GG; LIPIN-1 rs13412852 in which the predisposing genotype is CC or CT; SOD-2 rs4880 in which the predisposing genotype is TT; TM6SF2 rs58542926 in which the predisposing genotype is TT or CT.

3. Method according to either of claims 1 or 2 in which the risk of NAFLD is a risk associated with the development, evolution or progression of the disease.

4. Method according to any one of claims 1-3 in which the risk of developing NAFLD is associated with the development of fibrosis of the liver, steatosis, non-alcoholic steatohepatitis, cirrhosis of the liver or liver cancer.

**Patentansprüche**

1. Verfahren zur Vorhersage eines erhöhten Risikos für nicht-alkoholische Fettlebererkrankung (NAFLD) in einem Individuum, welches den Schritt der Genotypisierung von SNP rs12603226 aufweist, ausgeführt auf einer Nukleinsäure, welche aus einer biologischen Probe des Individuums gewonnen wurde, und in welcher die Anwesenheit von allelischer Homozygotie TT auf ein erhöhtes Risiko für die Entwicklung von NAFLD hinweist.

2. Verfahren gemäss Anspruch 1, ferner aufweisend den Schritt der Genotypisierung von einem oder mehreren SNPs, welche mit NAFLD assoziiert sind, ausgewählt aus: KLF-6 rs3750861, in welchem der prädisponierte Genotyp CC ist; PNPLA-3 rs738409, in welchem der prädisponierte Genotyp CG oder GG ist; LIPIN-1 rs13412852, in welchem der prädisponierte Genotyp CC oder CT ist; SOD-2 rs4880, in welchem der prädisponierte Genotyp TT ist; TM6SF2 rs58542926, in welchem der prädisponierte Genotyp TT oder CT ist.

3. Verfahren gemäss einem der Ansprüche 1 oder 2, in welchem das Risiko für NAFLD ein Risiko ist, welches mit der Entwicklung, Evolution oder Progression der Erkrankung assoziiert ist.

4. Verfahren gemäss einem der Ansprüche 1-3, in welchem das Risiko zur Entwicklung von NAFLD mit der Entwicklung von Leberfibrose, Steatose, nicht-alkoholischer Steatohepatitis, Leberzirrhose oder Leberkrebs assoziiert ist.

**Revendications**

1. Méthode pour prédire un risque accru de la stéatose hépatique non alcoolique (SHNA) chez un individu, comprenant l'étape de génotypage SNP rs12603226 effectué sur un acide nucléique obtenu à partir d'un échantillon biologique de l'individu et dans lequel la présence de l'homozygotie allélique TT indique un risque accru de développement de SHNA.

2. Méthode selon la revendication 1, comprenant en outre l'étape de génotypage d'un ou plusieurs SNPs associés à la SHNA sélectionnés parmi : KLF-6 rs3750861, où le génotype prédisposé est CC ; PNPLA-3 rs738409, où le génotype prédisposé est CG ou GG ; LIPIN-1 rs13412852, où le génotype prédisposé est CC ou CT ; SOD-2 rs4880, où le génotype prédisposé est TT ; TM6SF2 rs58542926, où le génotype prédisposé est TT ou CT.

3. Méthode selon la revendication 1 ou 2, dans laquelle le risque pour la SHNA est un risque associé au développement, à l'évolution ou à la progression de la maladie.

4. Méthode selon l'une des revendications 1 à 3, dans laquelle le risque de développer une SHNA est associé au développement de la fibrose hépatique, de la stéatose, de la stéatohépatite non alcoolique, de la cirrhose du foie ou du cancer du foie.

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5210015 A
- US 5538848 A
- US 5801115 A
- US 5118801 A
- US 5866336 A
- US 6117635 A
- US 20090197254 A
- US 20050042168 A1
- WO 1993009245 A1
- US 7904250 B
- WO 9215712 A
- US 5679524 A
- WO 9102087 A
- WO 9009455 A
- WO 9517676 A
- US 5302509 A
- US 5945283 A
- US 5605798 A
- WO 9322456 A
- WO 8910414 A, Wallace
- US 4988617 A
- US 6027889 A
- US 6268148 B
- US 5494810 A
- US 5830711 A
- US 6054564 A
- WO 9731256 A
- WO 0056927 A
- WO 9416101 A
- US 6955901 B
- CA 2400240
- WO 2007002204 A2
- US 7727723 B
- US 5270184 A
- US 5422252 A
- WO 0028082 A
- WO 0134790 A
- WO 0177317 A
- US 8093030 B
- US 6027923 A
- WO 1995003432 A1
- US 5399491 A
- EP 0629706 B1
- EP 0572417 A1

### Non-patent literature cited in the description

- **DUTKOWSKI, P. et al.** *Gastroenterology,* February 2015, vol. 148 (2), 307-23
- **BRUNT.** *Nat. Rev. Gastroenterol. Hepatol.,* 2010, vol. 7, 195
- **ANSTEE, Q.M. ; DAY, C.P.** *Nat. Rev. Gastroenterol. Hepatol.,* November 2013, vol. 10 (11), 645-55
- **MASUOKA, H.C. ; CHALASANI, NO.** *Ann. No. Y. Acad. Sci.,* 2013, vol. 1281, 106-12
- **FITZPATRICK, E. ; DHAWAN, A.** *World J. Gastroenterol.,* 2014, vol. 20, 10851-10863
- **NOBILI, V. et al.** *J. Pediatr. Gastroenterol. Nutr.,* May 2014, vol. 58 (5), 632-6
- **GUSELLA , J.F.** *Annual Review of Biochemistry,* July 1986, vol. 55, 831-854
- **VASAN, R.S.** *Circulation,* 16 May 2006, vol. 113 (19), 2335-62
- **DONGIOVANNI, P. et al.** Genetic Predisposition in NAFLD and NASH: Impact of Severity of Liver Disease and Response to Treatment. *Curr. Pharm. Des.,* September 2013, vol. 19 (29
- **NOBILI. V. et al.** *J. Pediatr. Gastroenterol. Nutr.,* May 2014, vol. 58 (5), 632-6
- **SOOKOIAN, S. et al.** *Hepatology,* February 2015, vol. 61 (2), 515-25
- **SCHNABL, B. et al.** *Int. J. Clin. Exp. Pathol.,* 2011, vol. 4 (7), 661-6
- **COLE et al.** *BMJ,* 06 May 2000, vol. 320 (7244), 1240-3
- **CHEN, X. ; SULLIVAN, P.F.** *Pharmacogenomics J.,* 2003, vol. 3 (2), 77-96
- **MARNELLOS, G.** *Curr. Opin. Drug Discov. Devel.,* May 2003, vol. 6 (3), 317-21
- **CHEN.** *Pharmacogenomics J.,* April 2003, vol. 3 (2), 197-205
- **BARISIC I et al.** *Appl. Microbiol. Biotechnol.,* January 2015, vol. 99 (1), 413-23
- **WARTELL et al.** *Nucl. Acid Res.,* 1990, vol. 18, 2699-2706
- **BONNEAU, J. ; HAYDEN, M.** *Methods Mol. Biol.,* 2014
- **KUHN, D.N.** *Electrophoresis,* January 2005, vol. 26 (1), 112-25
- **LAN, J.H. et al.** *Hum. Immunol.,* March 2015, vol. 76 (2-3), 166-75

- **MIKKELSEN, M. et al.** *Forensic Sci. Int. Genet.,* September 2014, vol. 12, 30-7
- *PLoS One.,* 24 July 2013, vol. 8 (7
- **CLARKE, A.C. et al.** *BMC Genomics,* 25 January 2014, vol. 15, 68
- *Mol. Biosyst.,* May 2014, vol. 10 (5), 970-1003
- **FREITAS, R.G. ; CAMPANA, E.M. ; POZZAN, R. ; BRANDÃO, A.A. ; BRANDÃO, A.P. ; MAGAL-HÃES, M.E. ; SILVA, D.A. ; ARQ. BRAS.** *Cardiol.,* June 2015, vol. 104 (6), 468-474
- Hamilton (ON). BC Decker, 2003
- **VALENTI et al.** *Hepatology,* April 2010, vol. 51 (4), 1209-17
- **KLEINER, D.E. et al.** *Hepatology,* 2005, vol. 41, 1313-21

SEQUENCE LISTING

<110> Orga Bio Human S.r.1.

<120> SNP rs12603226 come marcatore predittivo per 1a NAFLD.

<130> PCT-13588

<160> 4

<170> PatentIn version 3.5

<210> 1
<211> 17
<212> DNA
<213> synthetic

<400> 1
cccagccaaa tttatga        17

<210> 2
<211> 17
<212> DNA
<213> synthetic

<400> 2
cccagccaat tttatga        17

<210> 3
<211> 17
<212> DNA
<213> synthetic

<400> 3
caggcgtgag ccactgt        17

<210> 4
<211> 29
<212> DNA
<213> synthetic

<400> 4
gacaaatcat actgcttaaa agtgggttt        29